# EUROPÄISCHE PATENTSCHRIFT

(11) **EP 2 680 770 B1**
(45) Veröffentlichungstag und Bekanntmachung des Hinweises auf die Patenterteilung: **10.06.2015**
(21) Anmeldenummer: 12701068.4
(22) Anmeldetag: 25.01.2012
(51) Int. Cl.: A61B 17/68, A61B 17/84, A61B 17/86

(54) **SPANNELEMENT ZUM FIXIEREN EINER KNOCHENFRAKTUR SOWIE SELBIGES AUFWEISENDE FIXIERUNGSVORRICHTUNG**
CLAMPING ELEMENT FOR SETTING A BONE FRACTURE AND SETTING DEVICE COMPRISING SAME
ÉLÉMENT DE SERRAGE POUR LA FIXATION D'UNE FRACTURE OSSEUSE ET DISPOSITIF DE FIXATION POURVU DUDIT ÉLÉMENT

(30) Priorität: 02.03.2011 DE 102011001018
(43) Veröffentlichungstag der Anmeldung: 08.01.2014
(73) Patentinhaber: Hipp Medical AG, 78600 Kolbingen (DE)
(72) Erfinder: WAIZENEGGER, Markus, 78600 Kolbingen (DE)
(74) Vertreter: Kuhnen & Wacker
(86) Internationale Anmeldenummer: PCT/EP2012/000330
(87) Internationale Veröffentlichungsnummer: WO 2012/116773

(56) Entgegenhaltungen:
- EP-A1- 0 498 709
- WO-A2-2008/006098
- WO-A2-2008/051806
- US-A1- 2008 154 312

## Beschreibung

Die Erfindung betrifft ein Spannelement zum Fixieren einer Knochenfraktur nach Anspruch 1 sowie eine Fixierungsvorrichtung mit einem derartigen Spannelement nach Anspruch 6.

Bei unzureichender Ruhigstellung einer Knochenfraktur kann eine Kallusbildung, d.h. eine schwielenartige Verdickung der Bruchenden aus überwachsendem Knochengewebe, auftreten. Um eine solche indirekte Frakturheilung über einen Kallus zu vermeiden, werden Knochenplatten verwendet, die auf der Außenfläche eines gebrochenen Knochens aufgebracht und befestigt werden, damit die Bruchstelle während des Heilungsprozesses fixiert ist.

Für derartige Anwendungen zur Versorgung von Knochenfrakturen sind aus dem Stand der Technik zumeist starre, flächig ausgebildete, metallische Knochenplatten mit Bohrungen bekannt, die an den gegenüberliegenden Seiten einer Fraktur verschraubt werden. Zur Befestigung solcher Fixierungen werden üblicherweise sogenannte Corticalisschrauben verwendet. Diese werden in das äußere Knochengewebe, die sogenannte Corticalisschicht, welche die höchste Festigkeit des Knochens aufweist, eingeschraubt. Die Fixierung stellt sicher, dass sich die Bruchenden nicht gegeneinander bewegen können und sich neugebildetes Knochengewebe belastungsfrei anlagern kann.

Zudem wird angenommen, dass der Heilungsprozess einer Fraktur günstig beeinflusst werden kann, wenn eine Kompression auf die zusammengefügte Bruchstelle aufgebracht wird. Hierdurch wird eine besonders enge Adaption, d.h. ein geringes Spaltmaß, über welches die Bruchenden wieder aufeinander zu wachsen, bewirkt.

Um diese gewünschte Funktion zu erzielen, offenbart die die Offenlegungsschrift US 2008/0154312 A1 eine zweigeteilte Knochenplatte, deren Hälften sich mittels einer axialen Führung aufeinander zu bewegen können. Zur Herstellung einer Kompression ist in verschiedenen Ausführungsformen ein bandförmiges Elastomerelement vorgesehen, das die Hälften umspannt.

Die Offenlegungsschrift US 2007/0293863 A1 beschreibt eine Knochenbrücke mit zwei plattenförmigen Elementen zur Aufnahme von Knochenschrauben, die von einem elastischen Kabel durchzogen sind.

Die Offenlegungschrift US2008154312 A1 offenbart ein weiteres Spannelement für Knochenfrakturen.

Derartige einfache Konstruktionen von elastischen Knochenplatten bzw. -brücken sind mit dem Nachteil behaftet, dass die Vorspannung nicht nachjustiert werden kann. Gegebenenfalls kann durch eine gestaffelte Auswahl an elastischen Elementen mit unterschiedlicher Länge oder Spannungs-Dehnungs-Kennlinien eine stufenweise Anpassung bereitgestellt werden. Eine exakte Anpassung der Vorspannung hinsichtlich Maßtoleranzen von Befestigungspunkten ist jedoch nicht möglich.

Eine definierte Vorspannung ist bei der Abstimmung von mehrfachen, insbesondere beidseitig opponierenden, Fixierungen sowie bei indifferenten Bruchufern von Bedeutung, um über den gesamten Heilungsprozess eine exakte Ausrichtung der Bruchenden zueinander zu gewährleisten.

Demzufolge müssen bei der Anwendung obengenannter Vorrichtungen die Befestigungsmittel in einem operativen Eingriff äußerst positionsgetreu in den Knochen eingebracht werden. Zudem ist in der Praxis der Abstand der Befestigungsmittel, bedingt durch den Verlauf der Frakturlinien und der anatomischen Begebenheiten, nicht immer frei wählbar.

Des Weiteren ist ein System mit einem elastischen Wirkungsprinzip, d.h. mit einer ausgeprägten Spannungs-Dehnungs-Kennlinie, stets mit folgendem Nachteil behaftet:
Das Verhältnis zwischen der Vorspannung, die für eine effektive Lagefixierung gegenüber äußerlichen Krafteinwirkungen erforderlich ist und derjenigen Kraft, die aufgrund derselben Vorspannung zur Adaption auf die Bruchenden wirkt, ist direkt von der Spannungs-Dehnungs-Kennlinie des eingesetzten elastischen Elements abhängig. Das heißt mit anderen Worten, dass für eine wirksame Fixierung von Bruchstellen, auf die anatomisch bedingt eine große Hebelkraft bei Körperbewegungen wirkt, wie z.B. Röhrenknochen an Armen und Beinen, eine entsprechend hohe Vorspannung erforderlich ist, um eine Auslenkung der Bruchstelle durch potentielle Krafteinwirkungen sicher zu verhindern.

Diese zweckbedingte Vorspannung eines elastischen Elements übt jedoch permanent einen gegebenenfalls ungünstig hohen Druck auf das Knochengewebe der Bruchfläche aus.

Elastische Elemente mit einer entsprechend kurzen und steilen Spannungs-Dehnungs-Kennlinie weisen hierbei günstigere Eigenschaften hinsichtlich der Vorspannung zur Kompression und der Eignung zur Lagefixierung auf. Sie sind jedoch wiederum sensibler gegenüber Maßtoleranzen der Befestigungspunkte. Starre Knochenplatten entkoppeln diese beiden Eigenschaften, wobei eine gewünschte Kompression der Bruchenden ausbleibt.

Der Erfindung liegt daher die Aufgabe zugrunde, ein Spannelement für eine Fixierungsvorrichtung bereitzustellen, das eine hohe Lagefixierung gegenüber äußeren Kräften und eine einstellbare Kompression der Fraktur bereitstellt, die auf durch die Fraktur oder anatomisch bedingte Befestigungspunkte individuell angepasst werden kann und die dennoch besonders kostengünstig herstellbar ist, um den Anwendungsbereich kompressionserzeugender Fixierungen zu erweitern. Des Weiteren ist es eine Aufgabe, eine Fixierungsvorrichtung bereitzustellen, bei der das Spannelement angewendet wird.

Die Aufgabe wird durch das Spannelement gemäß Anspruch 1 sowie durch die Fixierungsvorrichtung gemäß Anspruch 6 gelöst.

Das erfindungsgemäße Spannelement weist eine flexible und im Wesentlichen nicht plastisch verformbare Endlosschleife, innerhalb der wenigstens zwei Befestigungsmittel, vorzugsweise Cortikalisschrauben, hindurchsteckbar sind, und ein Streckelement mit einer veränderbaren Länge auf, das in die Endlosschleife derart einsetzbar und innerhalb der Endlosschleife derart einspreizbar ist, dass zum Aufbringen von aufeinander zu gerichteten Kräften auf die Befestigungsmittel vermittels des Streckelements eine vorbestimmte Zugspannung über den Umfang der Endlosschleife erzeugbar ist.

Durch ein Verlängern des Streckelements erfährt die Endlosschleife bei gleichbleibendem Umfang eine Querkontraktion und bewirkt somit eine aufeinander zu gerichtete Vorspannung zwischen den umschlossenen Befestigungsmitteln. Da die Endlosschleife relativ unelastisch ist, d.h. eine kurze und steile Spannungs-Dehnungs-Kennlinie aufweist, kann auch bei einer wahlweise gering eingestellten Vorspannung, eine sichere Lagefixierung gegenüber äußerlichen Krafteinwirkungen gewährleistet werden. Somit ist die Kompression annähernd unabhängig von den anatomischen Hebelverhältnissen an der Bruchstelle individuell auf die Bruchfläche und das betroffene Knochengewebe einstellbar.

Aufgrund des einfachen Aufbaus des Spannelements kann die Fixierungsvorrichtung besonders kostengünstig hergestellt werden. Dadurch erweitert sich der Anwendungsbereich kompressionserzeugender Fixierungen zur Beschleunigung des Heilungsprozesses einerseits hinsichtlich des potenziellen Patientenkreises und andererseits auch auf verhältnismäßig unkritische Knochenfrakturen.

Die erfindungsgemäße Fixierungsvorrichtung weist wenigstens ein Spannelement und wenigstens zwei Befestigungsmittel, vorzugsweise Cortikalisschrauben, auf, die im Bereich der Bruchenden in den Knochen einbringbar sind, wobei vermittels des Streckelements eine vorbestimmte Zugspannung zwischen den von der Endlosschleife umschlossenen Befestigungsmitteln erzeugbar ist.

Die Erfindung sieht vor, die Kompression an einer Bruchstelle mittels der gespannten Endlosschleife herzustellen. Die Endlosschleife umläuft zum einen die Befestigungsmittel, die in den gegenüberliegenden Bruchenden eingebracht sind und zum anderen zwei gegenüberliegende Enden des Streckelements, das zwischen den Befestigungsmitteln verläuft. Da die Spannung der Endlosschleife über die Längenverstellung des Streckelements verstellbar ist, können Toleranzen bezüglich des Abstands der Befestigungsmittel ausgeglichen und die Kompression nachjustiert werden.

Vorteilhafte Weiterbildungen sind Gegenstand der abhängigen Ansprüche.

Gemäß einer Ausführungsform kann die Endlosschleife aus einem drahtartigen oder schnurartigen Material mit einem über dem Umfang der Endlosschleife im Wesentlichen konstanten Querschnitt bestehen. Somit ist eine flexible Ausbildung der Endlosschleife und eine gleichmäßige Flächenbelastung im Schleifenquerschnitt über den Umfang gewährleistet.

Gemäß einer Ausführungsform kann weiterhin das Streckelement derart zwischen den zwei Befestigungsmitteln anordnenbar sein, dass die Endlosschleife in zwei im wesentlichen gleich große Abschnitte untergliedert ist, wobei jeweils das eine Befestigungsmittel von einem vom Streckelement am weitesten entfernten Schleifenabschnitt zumindest teilweise umfasst ist. Somit verteilt sich die Spannung gleichmäßig auf die Schleifenabschnitte, und das Streckelement nimmt eine sichere Position bezüglich eines Verrutschens desselben in Umfangsrichtung der Endlosschleife ein.

Bei einer Ausführungsform kann das Streckelement zwei Schieber mit einem Gewindeabschnitt und eine dazwischen angeordnete Gewindehülse umfassen, wobei vorzugsweise einer der Schieber einen Linksgewindeabschnitt und der andere Schieber einen Rechtsgewindeabschnitt aufweist, und die Gewindehülse komplementär zu diesen ein Linksgewinde einerseits und ein Rechtsgewinde andererseits aufweist. Auf diese Weise kann die Längenverstellung bzw. die Justierung der Kompression besonders einfach durch ein Verdrehen der stets mittig verbleibenden Gewindehülse vorgenommen werden.

Bei einer Ausführungsform kann weiterhin der Schieber einen kreisbogenförmigen Abschnitt mit einer umfangsseitigen Nut zur Führung der Endlosschleife aufweisen, vermittels der die Endlosschleife vor einem Abrutschen von dem Schieber gesichert ist. Somit wird ein Abrutschen der Schieber des Streckelements in tangentialer Richtung des Endlosschleifenquerschnitts verhindert.

Die erfindungsgemäße Fixierungsvorrichtung kann weiterhin wenigstens eine Spannhülse, in der ein Befestigungsmittel aufnehmbar ist aufweisen, wobei die Spannhülse wenigstens teilweise von der Endlosschleife umschlossen wird. Die Spannhülse bildet einen Aufnahmeabschnitt zur Aufnahme der Endlosschleife an dem Befestigungsmittel. Durch die separate Ausgestaltung eines Hülsenelements und dem Befestigungsmittel ist die Verwendung von genormten und kostengünstig erhältlichen Befestigungsmitteln möglich.

In einer weiteren Ausführungsform kann die Spannhülse (2) zwei stirnseitige Kröpfungen umfassen. Somit kann ein Abspringen der Endlosschleife in axialer Richtung des Spannkopfs verhindert werden.

Gemäß einer beispielgebenden Ausführungsform kann die Spannhülse wenigstens eine Nut zur Führung der Endlosschleife aufweisen, vermittels der die Endlosschleife vor einem Verrutschen in axialer Richtung der Spannhülse gesichert ist.
An der Umfangsfläche der Spannhülse können mehrere parallel verlaufende Nuten ausgeprägt sein, in denen mehrere Endlosschleifen nebeneinander geführt werden können.

Somit wird ein Verknüpfungspunkt bereitgestellt, durch den die Kombination mehrerer Spannelemente, beispielsweise auch solcher mit verschiedener Abmessung und Ausrichtung, ermöglicht wird. Die erfindungsgemäße Fixierungsvorrichtung kann daher auf einfache Weise an die Struktur eines Bruchs, insbesondere einer Trümmerfraktur, sowie an die lokalen anatomischen Begebenheiten individuell angepasst werden.

Gegenüber einer Vielzahl einzelner herkömmlicher Knochenplatten lässt sich durch die Verknüpfung mehrerer Spannelemente die Anzahl der erforderlichen Befestigungspunkte verringern. Dies wirkt sich besonders bei kleinen Knochenfragmenten vorteilhaft aus und reduziert zudem den operativen Aufwand erheblich. Gegenüber individuell angefertigten Knochenplatten kann durch die modulare Konfigurationsmöglichkeit eine schnelle Versorgung von komplizierten Frakturen erreicht werden.

Der Heilungsprozess einer Knochenfraktur kann weiterhin begünstigt werden, wenn die stützende Corticalisschicht des Knochens nach einer gewissen Zeit Belastungen ausgesetzt wird, um wieder eine tragende Funktion zu übernehmen, so wie dies beispielsweise auch nach der Abnahme einer Gipsschienung der Fall ist. Bei einer herkömmlichen Fixierung wird hingegen ein Teil der tragenden Funktion dauerhaft durch die starre Knochenplatte übernommen.

Gemäß eines Aspekts der Erfindung kann die Fixierungsvorrichtung Elemente aus einem resorbierbaren Kunststoff, d.h. einem Material, das von dem umgebenden Körpergewebe abgebaut wird, umfassen. Resorbierbare Kunststoffe weisen jedoch relativ spröde Materialeigenschaften auf. Sie sind daher nur bedingt zur Ausbildung von Bereichen geeignet, die mechanischen Belastungen ausgesetzt sind.

Bei einer Ausführungsform kann demnach die Fixierungsvorrichtung wenigsten eine Spannhülse aufweisen, die aus einem resorbierbaren Kunststoff ausgebildet ist. Durch die Resorption der Spannhülse entsteht ein Spiel zwischen dem Befestigungsmittel und der Endlosschleife, das zu einer Kraftentkopplung zwischen den Befestigungsmitteln führt. Die Kraftübertragung zwischen der Fixierungsvorrichtung und dem Knochen geht daher mit fortschreitender Resorption der Hülsenelemente verloren, wodurch die Belastungen auf den Knochen wieder ansteigen.

Bei einer weiteren Ausführungsform kann wenigstens eine Spannhülse radial in zwei Abschnitte aufgeteilt sein, wobei der radial innere Abschnitt oder der radial äußere Abschnitts aus einem resorbierbaren Kunststoff ausgebildet ist. Durch die Resorption einer der beiden Abschnitte erfolgt in der Fixierungsvorrichtung ebenso eine Kraftentkopplung zwischen den Befestigungsmitteln, wie vorstehend erwähnt. Wenn vorzugsweise ein äußerer Abschnitt aus einem Metall und ein innerer Abschnitt aus einem resorbierbaren Kunststoff besteht, verbleibt mittels der stirnseitigen Kröpfungen des äußeren Abschnitts noch eine lokale Eingrenzung gegen ein Abspringen der Endlosschleife. Hierdurch kann ein Lösen der Endlosschleife aus seiner Anordnung bzw. eine potenzielle Beeinträchtigen des angrenzenden Köpergewebes verhindert werden.

Gemäß einer beispielgebenden Ausführungsform können sich benachbarte Spannelemente derart überschneiden, dass das Befestigungsmittel oder die Spannhülse von den überschneidenden Spannelementen umschließbar ist. Somit sind Fixierungsvorrichtungen aus einer kettenförmigen oder einer sternförmigen Anordnung von Spannelementen realisierbar. Durch eine Kettenanordnung kann bei einer geringen Anzahl von Befestigungsmitteln eine Kompression zwischen mehreren Knochenfragmenten erzielt werden. Durch eine Sternanordnung von Spannelementen kann eine mehrdirektionale Kompression auf Knochenfragmente mit überkreuzenden Frakturlinien, wie z.B. bei einer Trümmerfraktur und insbesondere einer Keilfraktur, aufgebracht werden. Durch die Wahl der Anordnung von Spannelementen und der Verknüpfungspunkte lässt sich die Fixierungsvorrichtung individuell auf den Verlauf verschiedener Bruchufer ausrichten.

Weitere Ausführungsformen, Merkmale und Vorteile der Erfindung werden anhand der nachfolgenden Beschreibung von Ausführungsformen unter Bezugnahme auf die Figuren ersichtlich. Dabei zeigt:
- Fig. 1: eine perspektivische Ansicht einer erfindungsgemäßen Fixierungsvorrichtung;
- Fig. 2: eine perspektivische Ansicht eines Schiebers des Streckelements;
- Fig. 3: eine perspektivische Ansicht einer Spannhülse;
- Fig. 4: eine perspektivische Ansicht einer Kettenanordnung von zwei Spannelementen;
- Fig. 5: eine perspektivische Ansicht einer radial aufgeteilten Spannhülse mit einem resorbierbaren Abschnitt.

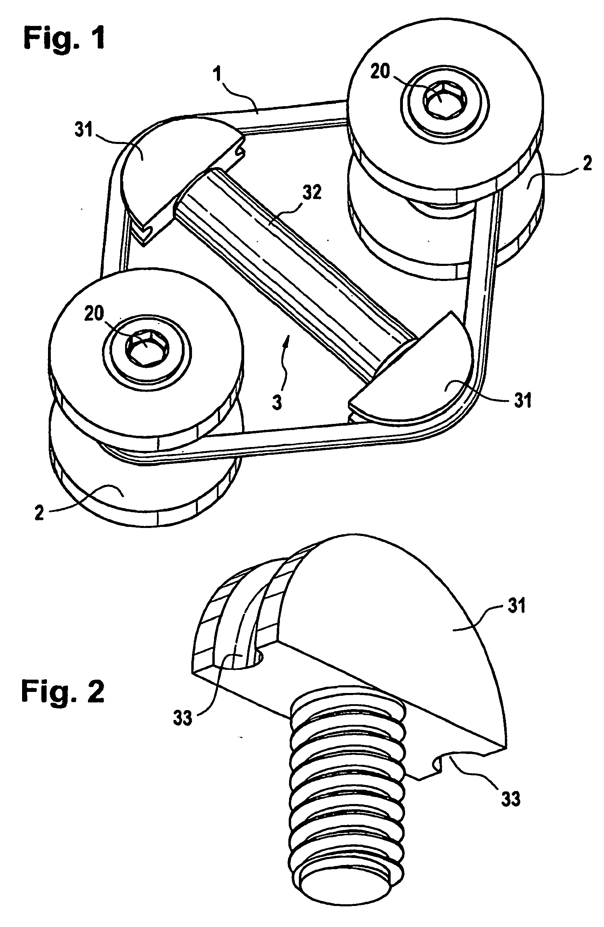

Fig. 1 zeigt eine Fixierungsvorrichtung mit zwei Spannhülsen 2 und ein Spannelement, das eine Endlosschleife 1 und ein Streckelement 3 aufweist. Die Endlosschleife 1 umläuft die beiden Spannhülsen 2 und wird von dem Streckelement 3 rautenförmig aufgespannt. In den beiden Spannhülsen 2 sind Befestigungsmittel 20 aufgenommen, welche in die gegenüberliegenden Bruchenden des betroffenen Knochens eingebracht werden. Das Streckelement 3 weist eine Gewindehülse 32 auf, welche zwei Schieber-Elemente 31 über zwei gegenläufige Gewindeabschnitte verbindet und verstellbar zueinander beabstandet.

Die Endlosschleife 1 wird beispielsweise aus einem Drahtseil gebildet. Da sie relativ unelastisch ist, d.h. eine steile Spannungs-Dehnungs-Kennlinie aufweist, sind wahlweise geringe Spannungen für eine sichere Fixierung ausreichend. Die permanente Kompression kann daher annähernd unabhängig der anatomischen Hebelverhältnisse an der Bruchstelle auf einen für die Bruchfläche bzw. das betroffene Knochengewebe vorteilhaften Bereich individuell eingestellt werden. Dabei werden ebenso Abweichungen des Abstands der Befestigungspunkte ausgeglichen, die durch Maßtoleranzen, die anatomische Gegebenheiten oder durch die Bruchuferverläufe bedingt sind.

Das Befestigungsmittel 20 kann ein chirurgisches Implantat wie z.B. ein Knochennagel, eine Knochenschraube und insbesondere eine Corticalisschraube sein, das in den Knochen eingebracht wird. Die Corticalisdicke der verschiedenen Knochen weicht hierbei je nach Knochen, z.B. an einem Oberschenkelknochen oder einem Kieferknochen, erheblich ab. Für die verschiedenen Anwendungen sind nach ISO 5835: 1991 oder ASTM: F 543-07 genormte Corticalisschrauben mit gestaffelten Durchmessern und Längen erhältlich.

Grundsätzlich sind hierbei jedoch keine festgelegten Vorgaben zwischen der Größe der Fixierungsvorrichtung und dem betreffenden Knochen vorgesehen. Allerdings ist eine proportionale Dimensionierung des Querschnitts der Endlosschleife oder des Streckelements 3 zu dem verwendeten Schraubensystem denkbar. Aus der praktischen Medizintechnik kann nährungsweise ein Zusammenhang zwischen dem Nenndurchmesser der Corticalis- bzw. Spongiosaschraube und dem Anwendungsbereich hergestellt werden, der in folgender Tabelle aufgeführt ist.

| Schraubensystem | Einsatzbereiche |
|---|---|
| HA 1,5 Corticalis | MGK-Chirurgie |
| HA 2,0 Corticalis | Cranio; Fußchirurgie; Handchirurgie |
| HA 2,5 Corticalis | Cranio; Fußchirurgie; Trauma |
| HA 3,5 Corticalis | Thorax; Unterer Rücken; Armchirurgie |
| HA 4,0 Corticalis | Thorax; Wirbelsäule; Hüft- und Beckenbereich |
| HA 4,5 Corticalis | Beinchirurgie; Rumpf; Fibula; Schulter |
| HA 5,0 Corticalis | Beinchirurgie; Tibia; Femur |
| HB 4,0 Spongiosa | abhängig vom Belastungsfall |
| HB 6,0 Spongiosa | abhängig vom Belastungsfall |

Die Wahl der Dimensionierung und Positionierung des Schraubensystems sowie der Fixierungsvorrichtung hängt zudem von der Frakturart (z.B. Querfraktur, Schrägfraktur) und dem Frakturort ab, wodurch sich verschiedene Belastungsfälle ergeben.

So kommen bei Schaftfrakturen zumeist Corticalissysteme zum Einsatz, da hier kein Spongiosaanteil vorhanden ist. Bei gelenknahen Frakturen kommen hingegen oftmals Spongiosasysteme zum Einsatz, da der Anteil der Spongiosa in diesem Bereich sehr hoch ist. Spongiosaschrauben weisen einen höheren Traganteil auf, da diese bei demselben Nenndurchmesser einen größeren Kerndurchmesser aufweisen. Bei Gelenkfrakturen, d.h. Frakturen mit Beteiligung der Gelenkfläche, werden in Abhängigkeit der lokalen anatomischen Bedingungen sowohl Cortikalissysteme als auch Spongiosasysteme verwendet. Ebenso können bei einer mehrfachen Fraktur beide Systeme eingesetzt werden.

Zudem können neben einer einfachen, d.h. monocorticalen Fixationsmöglichkeit auch bicorticale Systeme vorgesehen sein, bei denen die Schraube durch den Knochen hindurch an beiden corticalen Bereichen fixiert wird.

Nachdem die Befestigungsmittel 20 in den Knochen eingebracht und die Spannhülsen 2 fixiert sind, wird die Endlosschleife 1 um diese herum gelegt. Anschließend wird das Streckelement 3 senkrecht zu einer Linie durch die beiden Befestigungspunkte in der Endlosschleife 1 positioniert und eingespreizt. Durch Drehung der Gewindehülse 32 werden die gegenläufigen Gewindeabschnitte der Schieber-Elemente 31 beidseitig aus der Gewindehülse 32 herausgefahren, sodass das Streckelement 3 länger wird. Wenn die in Fig. 2 gezeigte umfangseitige Nut 33 der Schieber-Elemente 31 an der Endlosschleife 1 anliegt, wird diese unter Spannung gehalten. Um die Justierung der Längenverstellung zu erleichtern, ist an den Gewindeabschnitten der Schieber-Elemente 31 oder an einem Fensterdurchbruch in der Gewindehülse 32 beispielsweise eine Millimeteranzeige oder dergleichen vorgesehen.

Ferner kann das Befestigungsmittel 20 einen Abschnitt mit einer Spannhülse 2 umfassen. Die Spannhülse 2 weist nach oben und unten eine stirnseitige Kröpfung auf, die ein Abspringen der Endlosschleife 1 in axialer Richtung des Befestigungsmittels 20 verhindert. Die Spannhülse 2 ist beispielsweise separat ausgebildet und wird mit einer Corticalisschraube kombiniert, die in einer Durchgangsbohrung der Spannhülse 2 aufgenommen wird.

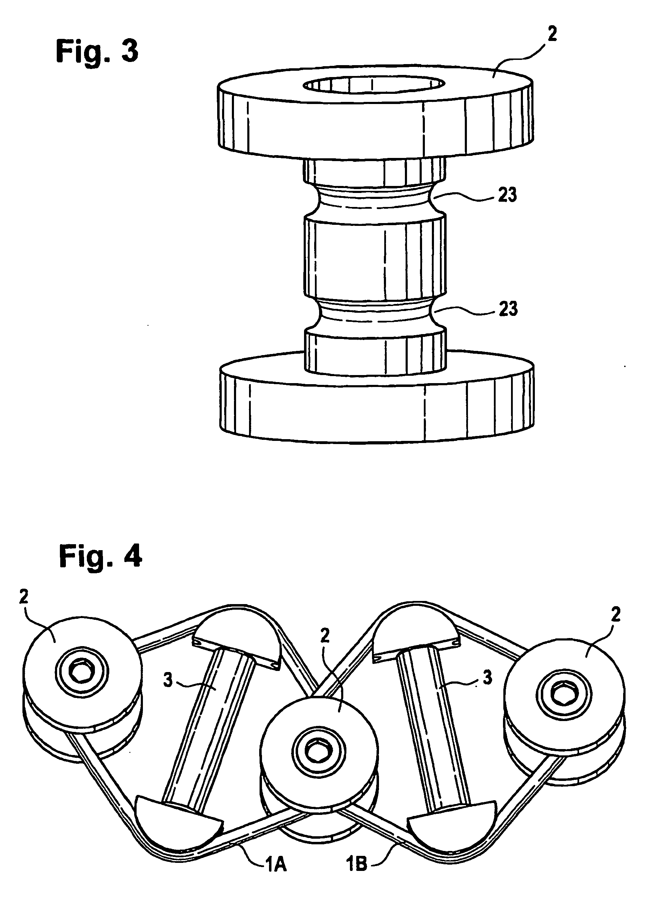

Fig. 3 zeigt eine Spannhülse 2 mit stirnseitigen Kröpfungen und zwei parallel verlaufenden Nuten 23 zur Führung von Endlosschleifen 1. Somit lassen sich, je nach Anzahl der Nuten 23, zwei oder mehrere verschiedene Spannelemente an einem Befestigungspunkt miteinander verknüpfen.

Durch diesen Aufbau kann die vorliegenden Fixierungsvorrichtung ebenso zu einer kettenförmigen Anordnung, wie in Fig. 4 dargestellt, zu einer sternförmigen Anordnung oder zu einer beliebigen Kombination aus Ketten- und Sternanordnungen erweitert werden. Je nach Anzahl der Nuten 23 in der Spannhülse 2 oder deren Breite zwischen den stirnseitigen Kröpfungen, kann z.B. eine Stemanordnung von drei oder mehr Spannelementen gebildet werden. Bei dieser Verknüpfung kann gegebenenfalls eine Spannhülse 2 ohne einer eingeführten Corticalisschraube oder dergleichen von den Endlosschleifen 1 umschlossen werden.

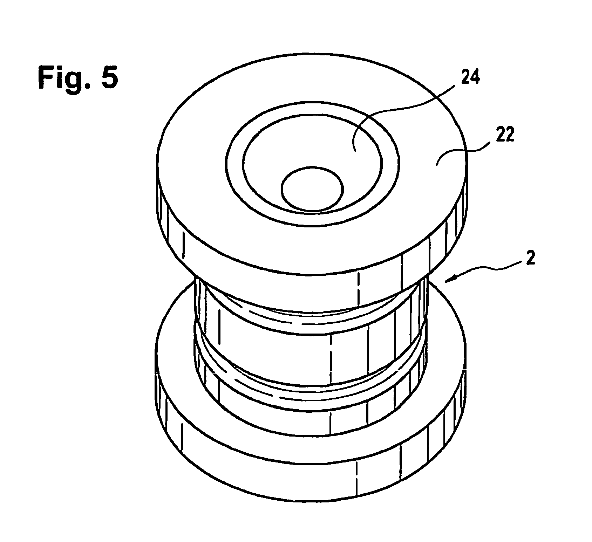

Die Spannhülse 2 in Fig. 5 umfasst einen radial inneren Abschnitt 24, in dem eine Corticalisschraube aufnehmbar ist. Der radial innere Abschnitt 24 wird durch eine Passung, vorzugsweise eine Presspassung, in den radial äußeren Abschnitt 22 der Spannhülse 2 eingefügt. Der radial innere Abschnitt 24 kann aus einem resorbierbaren Kunststoff und der radial äußere Abschnitt 22 kann aus einem Metall ausgebildet sein, oder umgekehrt.

Wenn z.B. der radial innere Abschnitt 24 resorbiert wird, entsteht in der Spannhülse 2 ein Leerraum um die Corticalisschraube herum, der dem Abschnitt 24 entspricht. Infolgedessen verschieben sich die Spannhülsen 2 und der Umfang zur Umspannung der beabstandeten Spannhülsen 2 verkleinert sich. Die Spannung der Endlosschleife 1 wird aufgehoben, sodass eine Kraftentkopplung zwischen den Befestigungsmitteln 20 stattfindet. Der Knochen wird somit wieder zunehmender Belastung ausgesetzt.

Die Elemente der Fixierungsvorrichtung werden aus biokompatiblen Materialen hergestellt und können einen Hybridaufbau, d.h. eine abschnittsweise Kombination mehrerer Werkstoffe umfassen.

Als Ausgangsmaterial für die Elemente der Fixierungsvorrichtung kommen beispielsweise Metalle aus der Gruppe: X42CrMo15, X100CrMo17, X2CrNiMnMoNNb21-16-5-3, X20Cr13, X15Cr13, X30Cr13, X46Cr13, X17CrNi16-2, X14CrMoS17, X30CrMoN15-1, X65CrMo 17-3, X55CrMo14, X90CrMoV18, X50CrMoV15, X 38CrMo V15, G-X 20CrMo13, X39CrMo17-1, X40CrMoVN16-2, X105CrMo17, X20CrNiMoS13-1, X5CrNi18- 0, X8CrNiS18-9, X2CrNi19-11, X2CrNi18-9, X10CrNi18-8, X5CrNiMo17-12-2, X2CrNiMo17-12-2, X2CrNiMoN25-7-4, X2CrNiMoN17-13-3, X2CrNiMo17-12-3, X2CrNiMo18-14-3, X2CrNiMo18-15-3; X 2 CrNiMo 18 14 3, X13CrMnMoN18-14-3, X2CrNiMoN22136, X2CrNiMnMoNbN21-9-4-3, X4CrNiMnMo21-9-4, X105CrCoMo18-2, X6CrNiTi18-10, X5CrNiCuNb16-4, X3CrNiCuTiNb12-9, X3CrNiCuTiNb12-9, X7CrNiAl17-7, CoCr20Ni15Mo, G-CoCr29Mo, CoCr20W15Ni, Co-20Cr-15W-10Ni, CoCr28MoNi, CoNi35Cr20Mo10, Ti1, Ti2, Ti3, Ti4, Ti-5Al-2,5Fe, Ti-5Al-2,5Sn, Ti-6Al-4V, Ti-6Al-4V ELI, Ti-3Al-2,5V (Gr9), 99,5Ti, Ti-12Mo-6Zr-2Fe, Ti-13,4Al-29Nb, Ti-13Nb-13Zr, Ti-15Al, Ti-15Mo, Ti- 15Mo-5Zr-3Al, Ti-15Sn, Ti-15Zr-4Nb, Ti-15Zr-4Nb-4Ta, Ti-15Zr-4Nb-4Ta-0,2Pd, Ti-29Nb-13Ta-4,6Zr, Ti-30Nb-10Ta-5Zr, Ti-35,5Nb-1,5Ta-7,1Zr, Ti-35Zr-10Nb, Ti-45Nb, Ti-30Nb, Ti-30Ta, Ti-6Mn, Ti-5Zr-3Sn-5Mo-15Nb, Ti-3Al-8V-6Cr-4Zr-4Mo, Ti-6Al-2Nb-1Ta-0,8Mo, Ti-6Al-4Fe, Ti-6Al-4Nb, Ti-6Al-6Nb-1Ta, Ti-6Al-7Nb, Ti-6Al-4Zr-2Sn-2Mo, Ti-8,4Al-15,4Nb, Ti-8Al-7Nb, Ti-8Al-1Mo-1V, Ti-11 Mo-6Zr-4Sn, in Betracht.

Ferner kommen Polymere aus der Gruppe: MBS, PMMI, MABS, CA, CTA, CAB, CAP, COC, PCT, PCTA, PCTG, EVA, EVAL, PTFE, ePTFE, PCTFE, PVDF, PVF, ETFE, ECTFE, FEP, PFA, LCP, PMMA, PMP, PHEMA, Polyamid 66, Polyamid 6, Polyamid 11 , Polyamid 2, PAEK, PEEK, PB, PC, PPC, PETP, PBT, MDPE, LDPE, HDPE, UHMWPE, LLDPE, PI, PAI, PEI, PIB, POM, PPO, PPE, PPS, PP, PS, PSU, PESU, PVC, PVC-P, PVC-U, ABS, SAN, TPE-U, TPE-A, TPE-E, PVDC, PVA, SI, PDMS, EPM, EP, UF, MF, PF, PUR, UP, PEBA, PHB, PLA, PLLA, PDLA, PDLLA, PGL, PGLA, PGLLA, PGDLLA, PGL-co-poly TMC, PGL-co-PCL, PDS, PVAL, PCL, Poly-TMC, PUR (linear), NiTi Superelastic, NiTi Shape Memory, in Betracht.

Des Weiteren kommen Keramiken aus der Gruppe: Al₂O₃ (Aluminiumoxid), Y-TZP (Zirkonoxidkeramik), AMC (Alumina Matrix Composite), HA (Hydroxilapatit), TCP (Tricalciumphosphat), Ceravital (Glaskeramik/Bioglas®), FZM/K (Zirkonoxid, teilstabilisiert), TZP-A (Zirkonoxidkeramik), ATZ (Alumina-toughened Zirconia), C799 (Aluminiumoxidkeramik), Schott 8625 (Transponderglas), in Betracht.

Darüber hinaus kommen Kombinationen hiervon in Betracht.

Die Erfindung lässt neben den gezeigten Ausführungsformen weitere Gestaltungsansätze zu.

So ist eine Ausführungsform der erfindungsgemäßen Fixierungsvorrichtung möglich, bei welcher die Spannhülsen 2 vollständig aus einem resorbierbaren Kunststoff bestehen. Wenn die Spannhülsen 2 durch das umgebende Körpergewebe resorbiert werden, entstehen entsprechende Leerräume. Wie obenstehend beschrieben, geht die Kraftübertragung der Fixierungsvorrichtung mit fortschreitender Resorption der Spannhülsen 2 verloren, wodurch die Belastung auf den Kochen wieder ansteigt.

Ferner ist es möglich an dem Streckelement 3 anstelle einer Gewindehülse eine axiale Führung zwischen den Schieber-Elementen 31 vorzusehen, an der eine Längenverstellung über ein Rastsystem, einen arretierbaren Exzenter, einen Kniehebel einen mittig gelagerten Lenkhebel oder ähnliche Mechanismen vorgenommen werden kann.

Überdies ist es auch möglich an dem Streckelement 3 einen Indikator, wie z.B. eine Skala, vorzusehen, die entweder basierend auf einer abgetragenen Länge, oder basierend auf einer Druckspannung, die an dem Streckelement erfasst wird, einen optischen Rückschluss zur Justierung einer geeigneten Spannung der Fixierungsvorrichtung zulässt.

Darüber hinaus ist es auch möglich, dass das Streckelement 3 durch eine Druckfeder oder ein ähnliches elastisches Element in Längsrichtung vorspannbar ist.

Mit der vorstehend diskutierten Erfindung wird ein Spannelement zum Fixieren einer Knochenfraktur bereitgestellt, dass eine flexible und im Wesentlichen nicht plastisch verformbare Endlosschleife aufweist. Innerhalb der Endlosschleife sind wenigstens zwei Befestigungsmittel, vorzugsweise Cortikalisschrauben, hindurchsteckbar sind. Das Spannelement weist weiterhin ein Streckelement mit einer veränderbaren Länge auf, das in die Endlosschleife derart einsetzbar und innerhalb der Endlosschleife derart einspreizbar ist. Mittels des Streckelements ist eine vorbestimmte Zugspannung über den Umfang der Endlosschleife erzeugbar ist, sodass dabei aufeinander zu gerichteten Kräfte auf die Befestigungsmittel aufgebracht werden. Weiterhin ist erfindungsgemäß eine Fixierungsvorrichtung zur Anwendung des Spannelements vorgesehen.

## Patentansprüche

1. Spannelement für Knochenfrakturen, aufweisend
eine flexible und im Wesentlichen nicht plastisch verformbare Endlosschleife (1), innerhalb der wenigstens zwei Befestigungsmittel (20), vorzugsweise Cortikalisschrauben, hindurchsteckbar sind, und
ein Streckelement (3) mit einer verstellbaren Länge, das in die Endlosschleife (1) derart einsetzbar und innerhalb der Endlosschleife (I) derart einspreizbar ist, dass
zum Aufbringen von aufeinander zu gerichteten Kräften auf die Befestigungsmittel (20) vermittels der Längenverstellung des Streckelements (3) eine vorbestimmte Zugspannung über den Umfang der Endlosschleife (1) erzeugbar ist.

2. Spannelement nach Anspruch 1, wobei die Endlosschleife (1) aus einem drahtartigen oder schnurartigen Material mit einem über dem Umfang der Endlosschleife (1) im Wesentlichen konstanten Querschnitt besteht.

3. Spannelement nach Anspruch 1 oder 2, wobei das Streckelement (3) derart zwischen den zwei Befestigungsmitteln (20) anordnenbar ist, dass die Endlosschleife (1) in zwei im Wesentlichen gleich große Abschnitte untergliedert ist, wobei jeweils das eine Befestigungsmittel (20) von einem vom Streckelement (3) am weitesten entfernten Schleifenabschnitt zumindest teilweise umfasst ist.

4. Spannelement nach wenigstens einem der Ansprüche 1 bis 3, wobei das Streckelement (3) zwei Schieber (31) mit einem Gewindeabschnitt und eine dazwischen angeordnete Gewindehülse (32) umfasst, wobei vorzugsweise einer der Schieber (31) einen Linksgewindeabschnitt und der andere Schieber (31) einen Rechtsgewindeabschnitt aufweist, und die Gewindehülse (32) komplementär zu diesen ein Linksgewinde einerseits und ein Rechtsgewinde andererseits aufweist.

5. Spannelement nach Anspruch 4, wobei der Schieber (31) einen kreisbogenförmigen Abschnitt mit einer umfangsseitigen Nut (33) zur Führung der Endlosschleife (1) aufweist, vermittels der die Endlosschleife (1) vor einem Abrutschen von dem Schieber (31) gesichert ist.

6. Fixierungsvorrichtung für Knochenfrakturen, aufweisend:
wenigstens ein Spannelement nach einem der Ansprüche 1 bis 5, und
wenigstens zwei Befestigungsmittel (20), vorzugsweise Cortikalisschrauben, die im Bereich der Bruchenden in den Knochen einbringbar sind,
wobei vermittels jedem des wenigstens einen Streckelements (3) eine vorbestimmte Zugspannung zwischen den von der dem betreffenden Streckelement (3) zugeordneten der wenigstens einen Endlosschleife (I) umschlossenen Befestigungsmitteln (20) erzeugbar ist.

7. Fixierungsvorrichtung nach Anspruch 6, weiterhin aufweisend wenigstens eine Spannhülse (2), in der ein Befestigungsmittel (20) aufnehmbar ist, wobei die Spannhülse (2) wenigstens teilweise von der Endlosschleife (1) umschlossen wird.

8. Fixierungsvorrichtung nach Anspruch 7, wobei die Spannhülse (2) zwei stirnseitige Kröpfungen umfasst.

9. Fixierungsvorrichtung nach Anspruch 7 oder 8, wobei die Spannhülse (2) wenigstens eine Nut (23) zur Führung der Endlosschleife (1) aufweist, vermittels der die Endlosschleife (1) vor einem Verrutschen in axialer Richtung der Spannhülse (2) gesichert ist.

10. Fixierungsvorrichtung nach einem der Ansprüche 7 bis 9, wobei wenigsten eine Spannhülse (2) aus einem resorbierbaren Kunststoff ausgebildet ist.

11. Fixierungsvorrichtung nach einem der Ansprüche 7 bis 10, wobei wenigstens eine Spannhülse (2) radial in zwei Abschnitte (22, 24) aufgeteilt ist und der radial innere Abschnitt (24) oder der radial äußere Abschnitt (22) aus einem resorbierbaren Kunststoff ausgebildet ist.

12. Fixierungsvorrichtung nach einem der Ansprüche 6 bis 11 mit wenigstens zwei Spannelementen, wobei sich benachbarte Spannelemente derart überschneiden, dass dasselbe Befestigungsmittel (20) oder dieselbe Spannhülse (2) von den. überschneidenden Spannelementen gemeinsam unischließbar ist.

## Claims

1. A clamping element for bone fractures, comprising
a flexible and substantially non-plastically deformable continuous loop (1), inside which at least two fixing means (20), preferably cortical screws, can be pushed through, and
an extension element (3) of alterable length that can be inserted into and is expandable inside the continuous loop (1) in such a manner that,
in order to apply forces directed towards each other to the fixing means (20), a predetermined tensile stress can be generated over the circumference of the continuous loop (1) by means of the regulation of the length of the extension element (3).

2. The clamping element according to claim 1, wherein the continuous loop (1) consists of a wire-like or cord-like material having a substantially constant cross-section over the circumference of the continuous loop (1).

3. The clamping element according to claim 1 or 2, wherein the extension element (3) can be arranged between the two fixing means (20) in such a manner that the continuous loop (1) is subdivided into two sections of substantially the same size, wherein the one fixing means (20), respectively, is at least partially enclosed by a loop section which is furthest from the extension element (3).

4. The clamping element according to anyone of claims 1 through 3, wherein the extension element (3) comprises two slides (31) with a threaded section, and a threaded sleeve (32) arranged therebetween, wherein preferably one of the slides (31) has a left-hand thread section and the other slide (31) has a right-hand section, and wherein, complementary to these, the threaded sleeve (32) has a left-hand thread on the one hand and a right-hand thread on the other hand.

5. The clamping element according to claim 4, wherein the slide (31) has an arc section with a circumferential groove (33) for guiding the continuous loop (1), by means of which the continuous loop (1) is secured against a slipping-off from the slide (31).

6. A fixation device for bone fractures, comprising:
at least one clamping element according to anyone of claims 1 through 5, and
at least two fixing means (20), preferably cortical screws, which can be inserted into the bone in the region of the ends of the fracture,
wherein by means of each of the at least one extension element (3) a predetermined tensile stress can be generated between the fixing means (20) which are enclosed by the at least one continuous loop (1) assigned to the corresponding extension element (3).

7. The fixation device according to claim 6, further comprising at least one clamping sleeve (2) inside which a fixing means (20) can be received, wherein the clamping sleeve (2) is enclosed at least partially by the continuous loop (1).

8. The fixation device according to claim 7, wherein the clamping sleeve (2) has two cranks at the end faces thereof.

9. The fixation device according to claim 7 or 8, wherein the clamping sleeve (2) comprises at least one groove (23) for guiding the continuous loop (1), by means of which the continuous loop (1) is secured against a slipping in the axial direction of the clamping sleeve (2).

10. The fixation device according to anyone of claims 7 through 9, wherein at least one clamping sleeve (2) is made of an absorbable synthetic material.

11. The fixation device according to anyone of claims 7 through 10, wherein at least one clamping sleeve (2) is radially divided into two sections (22, 24), and the radially inner section (24) or the radially outer section (22) is made of an absorbable synthetic material.

12. The fixation device according to anyone of claims 6 through 11, with at least two clamping elements, wherein adjacent clamping elements overlap each other in such a manner that the same fixing means (20) or the same clamping sleeve (2) can be jointly enclosed by the overlapping clamping elements.

## Revendications

1. Elément de serrage pour des fractures osseuses, présentant
une boucle sans fin (1) flexible et sensiblement non déformable plastiquement, dans laquelle au moins deux moyens de fixation (20), de préférence des vis à corticale, peuvent être enfichés, et
un élément étirable (3) avec une longueur réglable qui peut être inséré dans la boucle sans fin (1) et déployé à l'intérieur de la boucle sans fin (1) de telle manière que
pour l'application de forces dirigées les unes vers les autres sur les moyens de fixation (20) à l'aide du réglage de longueur de l'élément étirable (3), un effort de traction prédéterminé puisse être généré sur la périphérie de la boucle sans fin (1).

2. Elément de serrage selon la revendication 1, la boucle sans fin (1) se composant d'un matériau de type cordon ou de type fil métallique avec une section sensiblement constante sur la périphérie de la boucle sans fin (1).

3. Elément de serrage selon la revendication 1 ou 2, l'élément étirable (3) pouvant être agencé entre les deux moyens de fixation (20) de telle manière que la boucle sans fin (1) soit subdivisée en deux sections sensiblement de même grandeur, respectivement l'un moyen de fixation (20) étant compris au moins en partie par une section de boucle la plus éloignée de l'élément étirable (3).

4. Elément de serrage selon au moins l'une quelconque des revendications 1 à 3, l'élément étirable (3) comportant deux coulisseaux (31) avec une section filetée et une douille filetée (32) agencée au milieu, de préférence l'un des coulisseaux (31) présentant une section filetée gauche et l'autre coulisseau (31) une section filetée droite, et la douille filetée (32) présentant de manière complémentaire à celles-ci un filet gauche d'une part et un filet droit d'autre part.

5. Elément de serrage selon la revendication 4, le coulisseau (31) présentant une section en forme d'arc de cercle avec une rainure côté périphérie (33) pour le guidage de la boucle sans fin (1), à l'aide de laquelle la boucle sans fin (1) est protégée contre tout glissement du coulisseau (31).

6. Dispositif de fixation pour des fractures osseuses, présentant :
au moins un élément de serrage selon l'une quelconque des revendications 1 à 5, et
au moins deux moyens de fixation (20), de préférence des vis à corticale qui peuvent être introduites dans la zone des extrémités fracturées dans l'os,
un effort de traction prédéterminé entre les moyens de fixation (20) entourés par l'au moins une boucle sans fin (1), associés à l'élément étirable (3) concerné, pouvant être généré à l'aide de chacun de l'au moins un élément étirable (3).

7. Dispositif de fixation selon la revendication 6, présentant en outre au moins une douille de serrage (2), dans laquelle un moyen de fixation (20) peut être reçu, la douille de serrage (2) étant entourée au moins en partie par la boucle sans fin (1).

8. Dispositif de fixation selon la revendication 7, la douille de serrage (2) comportant deux coudes côté avant.

9. Dispositif de fixation selon la revendication 7 ou 8, la douille de serrage (2) présentant au moins une rainure (23) pour le guidage de la boucle sans fin (1), à l'aide de laquelle la boucle sans fin (1) est protégée contre tout glissement dans le sens axial de la douille de serrage (2).

10. Dispositif de fixation selon l'une quelconque des revendications 7 à 9, au moins une douille de serrage (2) étant réalisée en un plastique résorbable.

11. Dispositif de fixation selon l'une quelconque des revendications 7 à 10, au moins une douille de serrage (2) étant divisée radialement en deux sections (22, 24) et la section radialement intérieure (24) ou la section radialement extérieure (22) étant réalisée en un plastique résorbable.

12. Dispositif de fixation selon l'une quelconque des revendications 6 à 11, avec au moins deux éléments de serrage, des éléments de serrage contigus se coupant de telle manière que le même moyen de fixation (20) ou la même douille de serrage (2) puisse être entouré par les éléments de serrage se coupant ensemble.
